# EUROPÄISCHE PATENTANMELDUNG

(11) **EP 1 698 353 A1**
(43) Veröffentlichungstag der Anmeldung: **06.09.2006**
(21) Anmeldenummer: 06110623.3
(22) Anmeldetag: 03.03.2006
(51) Int. Cl.: A61L 2/20, A01M 17/00, A23B 9/18, A23B 9/20, A23L 3/3418

(54) **Verfahren und Vorrichtung zum Behandeln von Schüttgut mit Kohlendioxid**

(30) Priorität: 04.03.2005 CH 3832005
(71) Anmelder: Fraefel, Ramo, 9200 Gossau (CH)
(72) Erfinder: Fraefel, Ramo, 9200 Gossau (CH)
(74) Vertreter: Stocker, Kurt

(57) **Zusammenfassung**

Zur Schädlingsbekämpfung wird Schüttgut in einem Silo (2) mit Kohlendioxid behandelt. Das Silo (2) umfasst eine obere Befüllöffnung (3) und eine untere Austragsöffnung (4). Am Silo (2) angeordnet sind eine Gasleitung (15) mit Ventil (16) zum Zuführen von Kohlen-dioxid und eine Zirkulationsleitung (27) von einem oberen zu einem unteren An-schluss (28, 29) ans Siloinnere. An der Zirkulationsleitung (27) sind ein Zirkula-tionsgebläse (30) und eine Zirkulationsheizung (31) angeordnet. Am Anfang einer Be-handlung wird Schüttgut eingefüllt, die obere Befüllöffnung (3) gas-dicht verschlossen, anschliessend Kohlendi-oxid durch eine Gasleitung (15) ins Silo (2) einge-tragen und Luft durch eine am oberen Siloende angeschlossene mit der Umge-bung verbundene Ausgleichsleitung (21) abgelassen. Beim Eintragen von Kohlendioxid wird das Durch-strömen der Zirkulationsleitung (27) von unten nach oben durch ein Ventil (32) verhin-dert. Nach dem Befüllen des Silos (2) mit Kohlendioxid werden die Gasleitung (15) und die Ausgleichsleitung (21) gasdicht ver-schlossen. Zum Fördern von Gas durch die Zir-kulationsleitung (27) und das Schüttgut wird das Zirkulationsgebläse (30) einge-schaltet. Die Zirkulationsheizung (31) wird eingeschaltet, um das ge-förderte Gas und damit das Schüttgut zu erwärmen bis das Schüttgut auf Temperaturen erwärmt ist, wel-che über einer vorgegebenen minimalen Behandlungstemperatur liegen. Nach einer vorgegebenen Behandlungszeit wird das Kohlendioxid ausgetragen.

## Beschreibung

Die Erfindung bezieht sich auf Verfahren nach dem Oberbegriff des Anspruches 1 und auf Vorrichtungen nach dem Oberbegriff des Anspruches 7.

Verschiedenste Nahrungs- oder Futtermittel werden in der Form von körnigem Schütt-gut transportiert und gelagert. Die Schädlingsbekämpfung bei Nahrungs-, oder Futter-mitteln soll eine möglichst vollständige Abtötung der Schädlinge gewährleisten und es sollten keine Giftstoffe im Produkt verbleiben. Produkte mit einem Bio-Label dürfen nicht mit Giftstoffen behandelt werden. So wird beispielsweise Getreide in Lagersilos mit Kohlendioxid behandelt. Dabei müssen grosse Volumen über lange Zeiten mit CO² begast werden. Weil die gängigen Betonsilos bezüglich Gasdurchtritt nicht dicht sind, tritt viel CO² durch die Silowand aus. Auch wenn CO² nachgefüllt wird, kann nicht si-chergestellt werden, dass kein Sauerstoff im Schüttgut verbleibt. Einzelne Schädlinge können aufgrund von Restsauerstoff überleben. Es gibt auch Schädlinge, die bei genü-gend tiefen Temperaturen sehr lange ohne Sauerstoff überleben. Es muss daher eine lange Behandlungszeit gewählt werden. Das Einbringen von CO² in Lagersilos ist sehr aufwändig, ohne dass eine Garantie für eine genügende Schädlingsbekämpfung gege-ben werden kann.

Das europäische Patent EP 416 255 B1 beschreibt ein Begasungsverfahren bei dem der Gebäudeinnenraum einer Mühle gegen aussen abgedichtet, der Innenraum erwärmt und Kohlendioxid in den Innenraum eingebracht wird. Gemäss der Erfin-dung wird die Innenluft so lange auf einer Temperatur von 35 bis 50°C gehalten bis die Gebäudeinnenwände eine Temperatur von mindestens 27°C haben. Der Luftin-halt des Gebäudes wird mit einem Anteil an Kohlendioxid von mindestens 60% und einer Temperatur zwischen 30°C und 40°C ersetzt. Zur Behandlung werden ca. 2 Tage benötigt. Es hat sich gezeigt, dass diese Behandlung für Lagerräume mit Schüttgut nicht geeignet ist. Im Schüttgut überleben zu viele Schädlinge.

Aus der Schrift DE 43 08 585 A1 ist ein weiteres Begasungsverfahren bekannt, bei dem Kohlendioxid in ein abgedichtetes Gebäude eingetragen wird. Die Temperatur des Innen-raumes wird von einem Heizkörper in einem Bereich von 12°C bis 26°C gehalten. Über eine Gasleitung wird Kohlendioxid oder Stickstoff in den Innenraum eingetragen. Die Kohlendioxid-Konzentration wird erfasst. Mit einem Kohlendioxid-anteil von 80 Vol.-% wird eine Behandlungsdauer von 1 bis 3 Wochen vorgeschla-gen. Diese Behandlungsdauer ist für die Behandlung von Schüttgut sehr lange. Zu-dem überleben im Schüttgut zu viele Schädlinge.

Aus der DE 44 10 116 A1 ist ein Verfahren bekannt, bei dem in einer gasdichten Kammer mit Kunstgegenständen zuerst ein Unterdruck dann mit dem Einbringen von Kohlendioxid ein Überdruck und nach dem Messen des Restsauerstoffgehaltes gegebenenfalls diese beiden Schritte wiederholt werden. Um den Gasverbrauch zu vermindern, wird das abgesaugte Gas über eine Sauerstofftrennanlage geleitet und danach wieder in die Kammer geführt. Zur gleichmässigen Verteilung des Gases in der Kammer können Ventilatoren eingesetzt werden. Dieses Verfahren ist für Schüttgut nicht geeignet.

Das deutsche Patent DE 33 48 389 C2 beschreibt eine Begasung mit Metallphos-phid und Kohlendioxid. Wenn die Phosphinkonzentration bei der Eintragsöffnung eine maximal zulässige Konzentration überschreitet, wird ein geschlossener lufthal-tiger Gasstrom eingeschaltet, welcher durch das Gebiet führt, in dem Phosphin frei-gesetzt wird. Durch die damit verbundene Phosphinverdünnung kann eine Selbst-entzündung vermieden werden. Nach dem Eintragen des Phosphins wird die Gas-umwälzung beendet. Gemäss einer beschriebenen Ausführungsform wird Getreide durch eine Einstiegsluke in ein Silo eingefüllt. Die Einstiegsluke im Dach des Silos wird gasdicht verschlossen. Ein Gasumwälzgerät mit einem Ansauggebläse, einem Ansaugrohr und einem Ausgangsrohr ist so am Silo angeschlossen, dass Gas am oberen Ende des Silos angesaugt und in den unteren Teil des Getreides durch Gaseintrittsöffnungen eingetragen wird. Am Ansaugrohr ist ein ventilgesteuerter Luftansaugstutzen vorgesehen, durch den auch das Kohlendioxid zugeführt werden kann. Gegebenenfalls wird das Kohlendioxid durch einen gesonderten Stutzen in das Ansaugrohr eingetragen. Vom Ansaugrohr gelangt das Kohlendioxid durch das Ansauggebläse und das Ausgangsrohr ins Silo. Das Ansauggebläse kann nach einem festen Programm betrieben werden. Gegebenenfalls wird zur Steuerung des Ansauggebläses mindestens eine gemessene Phosphinkonzentration verwendet. Die Regelung der Kohlendioxidzuführung ist nicht beschrieben. Die beschriebene Lösung gewährleistet die Schädlingsbekämpfung nur beim Einsatz von Phosphin und ist daher für giftfreie Behandlungen ungeeignet. Die Vorrichtung ist nicht ge-eignet, um im Silo eine möglichst hohe Kohlendioxidkonzentration zu erzielen.

Das Patent EP 1 092 348 B1 beschreibt eine CO² Behandlung mit einem Druck von 20 bar. Mit zwei druckfesten Behandlungsbehältern, einem Zwischenbehälter und einem Verdichter wird ermöglicht, dass das Behandlungsgas annähernd vollständig wieder verwendbar ist. Die Vorrichtung und das Behandlungsverfahren ist aufgrund der hohen Drucke und der alternierend eingesetzten Behandlungsbehälter sehr aufwändig.

Die erfindungsgemässe Aufgabe besteht nun darin ein Verfahren und eine Vorrichtung zu finden, welche eine möglichst vollständige Schädlingsbekämpfung einfach und effi-zient ermöglichen.

Die Aufgabe wird durch die Merkmale der Ansprüche 1 und 7 gelöst. Die abhängigen Patentansprüche beschreiben bevorzugte Ausführungsformen.

Im Rahmen der Erfindung wurde erkannt, dass bei Schüttgut für eine erfolgreiche Schädlingsbekämpfung mit Kohlendioxid zwei Bedingungen erfüllt sein müssen. Erstens muss das Schüttgut im Wesentlichen frei von Sauerstoff und somit nur von Kohlendi-oxid umgeben sein. Zweitens muss im Wesentlichen das gesamte Schüttgut eine Tem-peratur in einem vorgegebenen Temperaturbereich aufweisen.

Um diese Bedingungen erfüllen zu können, wird das Schüttgut für die Behandlung in eine dichte Behandlungszelle, insbesondere in ein dichtes Silo eingefüllt. Das Volumen sollte nicht zu gross sein, weil sonst das Erreichen einer gewünschten Temperatur viel Zeit beansprucht. Es wurde erkannt, dass die Behandlung am bes-ten zwischen einem Transport und einer Lagerung durchgeführt wird, vorzugsweise vor, gegebenenfalls aber auch nach der Lagerung in einem Silo. Entsprechend sollte das Aufnahmevolumen der Behandlungszelle in der Grössenordnung der gängigen Transportvolumen liegen. Wenn Getreide mit Bahnwagen oder gegebe-nenfalls Lastwagen zum Silo geliefert wird, so sollte zumindest das halbe, insbe-sondere aber das gesamte Transportvolumen in der Behandlungszelle Platz finden. Vom Bahnwagen oder Lastwagen kann das Schüttgut beispielsweise über ein Vor-depot durch eine obere Befüllöffnung in ein Behandlungssilo eingebracht werden. Nach dem Einbringen des Schüttgutes wird die Befüllöffnung dicht verschlossen, vorzugsweise mit einem gasdichten Schieber, der insbesondere automatisch zu betätigen ist. Am untern Ende des Silos ist eine Austragsöffnung ebenfalls mit einem gasdichten Schieber verschlossen.

Die Dichtigkeit des Silos wird gegebenenfalls vor dem Eintragen von Kohlendioxid mit einem Testprogramm geprüft. Dazu kann der Siloinnenraum mit einem kleinen Überdruck beaufschlagt werden. Mit einer Drucküberwachung kann der Druckver-lauf über einen vorgegebenen Zeitraum überwacht werden. Bei einem genügenden Druckerhalt kann von der nötigen Dichtheit ausgegangen werden. Es versteht sich von selbst, dass nebst den gasdichten Schiebern auch alle Leitungen, die mit dem Siloinnern und der Umgebung oder einem anderen System verbunden sind, mittels gasdichter Ventile verschliessbar sein müssen.

Für die Schädlingsbekämpfung wird am Anfang einer CO² Behandlung über eine Gasleitung mit einem Anschluss am unteren Siloende Kohlendioxid ins Siloinnere eingetragen und am Ende der Behandlung wieder ausgelassen. Die Gasleitung führt durch ein erstes gasdichtes Schliessventil, welches beim Eintragen und beim Aus-lassen von Kohlendioxid geöffnet und sonst geschlossen ist. Am oberen Siloende ist eine Ausgleichsleitung über ein zweites gasdichtes Schliessventil mit dem Silo-innern verbunden. Beim Eintragen und beim Auslassen des Kohlendioxid wird das zweite gasdichte Schliessventil geöffnet, so dass beim Eintragen von Kohlendioxid durch die Ausgleichsleitung Luft austreten und beim Auslassen des Kohlendioxid Luft eintreten kann.

Beim Einfüllen des Kohlendioxid ist es vorteilhaft, wenn sich im Siloinnern keine turbulenten Gaszirkulationen ausbilden. Das Kohlendioxid soll das Silo langsam von unten nach oben füllen und dabei die Luft aus dem Silo verdrängen. Nach dem -Befüllen des Freiraums im Silo und somit auch des Freiraums im Schütt-gut werden das erste und das zweite gasdichte Schliessventil geschlossen. Der Zeitpunkt für das Schliessen kann über das eingebrachte CO² Volumen, über eine vorgegebene Befüllzeit oder auch durch Sensoren, die den Sauerstoff- bzw. Koh-lendioxidgehalt am oberen Siloende erfassen, bestimmt werden. Kohlendioxid wirkt bereits bei Vo-lumenanteilen unter 50% toxisch für die Schädlinge. Über einem Kohlendioxidanteil von 60% ist die Wirkung deutlich erhöht. Die toxische Wirkung nimmt bei Kohlen-dioxidanteilen über 80% nochmals klar zu und ist bei einem Kohlendioxidanteil über 90% optimal. Es wird ein möglichst hoher Kohlendioxidanteil angestrebt, weil dann aufgrund der hohen Toxizität die Schädlinge mit einer kurzen Behandlungsdauer weitgehend vernichtet werden können.

Für eine erfolgreiche Schädlingsbekämpfung wird das Schüttgut auf Temperaturen in einem vorgegebenen Temperaturbereich erwärmt. Es wäre möglich das Schüttgut schon vor dem Einbringen ins Silo zu erwärmen. Weil aber das Schüttgut während der Behandlungszeit die gewünschte Temperatur halten sollte, muss auch bei vor-gewärmtem Schüttgut eine Wärmezuführung vorgesehen werden, um das Absinken der Temperatur zu vermeiden.

Mit einer Warmgas-Strömung in vertikaler Richtung durch das Silo kann das Schüttgut erwärmt bzw. auf der gewünschten Temperatur gehalten werden. Um eine geschlossene Zirkulation zu erzielen, wird ausserhalb des Silos eine Zirkulations-leitung von einem oberen zu einem unteren Anschluss geführt. Die Zirkulationslei-tung umfasst ein Zirkulationsgebläse und eine Heizvorrichtung, so dass bei nach aussen gasdicht verschlossenem Silo vom Zirkulationsgebläse Gas durch die Zir-kulationsleitung und das Silo gefördert werden kann, wobei das Gas bei Bedarf von der Heizvorrichtung erwärmt wird. Das erwärmte Gas gibt beim Durchströmen des Schüttgutes Wärme ans Schüttgut ab. Um den Wärmeverlust minimal zu halten, wird das Silo und gegebenenfalls auch die Zirkulationsleitung zumindest teilweise isoliert. Das Gas darf beim Eintritt ins Schüttgut eine vorgegebene Temperatur nicht überschreiten, damit das Schüttgut beim Gaseintritt nicht über eine zulässige Tem-peratur erwärmt wird. Die meisten Nahrungsmittelschüttgüter sollten nicht über 40°C erwärmt werden. Bei unbearbeitetem Getreide würde die Keimfähigkeit verän-dert und Enzyme könnten zerstört werden. Die Tem peratur des Schüttgutes im Gaseintrittsbereich kann mit einem Temperatursensor überwacht werden. Mit einer Steuerung kann gewähr-leistet werden, dass die Heizleistung der Zirkulationshei-zung reduziert wird, wenn die Temperatur im Schüttgut über einen vorgegebenen Wert ansteigt. Gegebenen-falls wird die Zirkulationsheizung auch ganz ausgeschal-tet und das zirkulierende Gas zur Homogenisierung der Temperaturverteilung im Schüttgut eingesetzt.

Weil die Zirkulationsleitung beim Einfüllen des Kohlendioxid einen Bypass zum Silo bildet, muss mit einem Einwegventil in der Zirkulationsleitung verhindert werden, dass das eingebrachte Kohlendioxid anstatt das Silo zu befüllen direkt durch die Zirkulationsleitung zum oberen Siloende gelangt und dort entweicht. Das Einweg-ventil lässt in der Zirkulationsleitung nur eine Strömung von oben nach unten zu. Es verschliesst die Zirkulationsleitung, wenn der Druck unterhalb des Ventils grösser ist als oberhalb des Ventils. Es wäre auch möglich ein gesteuertes Ventil einzuset-zen, das beim Einfüllen des Kohlendioxids geschlossen, und insbesondere nur bei einge-schaltetem Zirkulationsgebläse offen, ist.

Die Beheizung des Schüttgutes wird entsprechend der Grösse bzw. Form des Silos optimiert. So können gegebenenfalls mit weiteren Zirkulationsleitungen vertikale Siloabschnitte getrennt mit zirkulierendem Gas behandelt werden. Es ist auch mög-lich eine Zirkulationsleitung mit mehr als zwei Anschlussstellen vorzusehen. Zwei untere Anschlussstellen auf verschiedenen Höhen sind zwar möglich, haben aber den Nachteil, dass bei lediglich einer Zirkulation mit Durchflussregulierungen gewähr-leistet werden muss, dass an beiden Stellen Gas eintritt. Gegebenenfalls wird die Eintritt-stelle phasenweise gewechselt.

Bei der Behandlung von körnigem Schüttgut mit durchströmendem Gas besteht die Gefahr, dass Kurzschluss-Strömungskanäle mit einem hohen Luftdurchsatz entstehen und die Temperaturverteilung nicht gleichmässig bzw. nicht homogen ist. Darum wird das Zirkulationsgebläse entsprechend dem Siloquerschnitt so dimensioniert bzw. be-trieben, dass möglichst keine grossen Strömungskanäle durch das Schüttgut entstehen. Dazu wird eine kleine Durchströmungsgeschwindigkeit gewählt. Der untere Anschluss der Zirkulationsleitung weist vorzugsweise eine Vielzahl kleiner Öffnungen auf, so dass das Zirkulationsgas über einen Flächenbereich verteilt eintritt.

An den Eintrittsstellen des unteren Anschlusses der Zirkulationsleitung wird ein zu-lässiger maximaler Temperaturwert schnell erreicht, so dass die Heizleistung der Zirkulationsheizung reduziert werden muss. Bei grossen Schüttgutmengen bzw. grossen Silos ist es daher für die Beschleunigung der Behandlung vorteilhaft, wenn nebst der Beheizung mit dem Zirkulationsgas eine Wärmeübertragung von den Si-lowänden ans anliegende Schüttgut vorgesehen wird. Die Silowandbeheizung er-folgt vorzugsweise elektrisch. Es eignen sich Heizmatten oder Heizbänder. Weil der obere Bereich des Silos bei kleineren Chargen nicht befüllt ist, wird gegebenenfalls am unteren Bereich des Silos mehr Heizleistung installiert als am oberen. Es ist auch möglich die Wandheizung auf verschiedenen Niveaus getrennt einschalten zu können, so dass im Wesentlichen die Silowand bis zum aktuellen Füllstand beheizt wird, wobei der Füllstand erfasst oder eingegeben werden muss. Um eine Über-hitzung des Schüttgutes an den Silowänden zu vermeiden, können mittels Tempe-ratursensoren Schüttguttemperaturen bei den Silowänden erfasst und zur Steue-rung der Wandbeheizung eingesetzt werden.

Die erfinderische Lösung ermöglicht es eine im Wesentlichen vollständige Schädlings-bekämpfung einfach und effizient durchzuführen. Das Kohlendioxid wird so eingefüllt, dass es im Wesentlichen zu allen Freiräumen im Silo und insbesondere im Schüttgut -gelangt. Die Zirkulationsströmung mit der Beheizung des zirkulierenden Gases bringt im Wesentlichen alles Schüttgut auf eine für die Schädlingsbehandlung optimale Tempe-ratur. Mit der Silowand-Beheizung wird die gewünschte Temperatur noch schneller und homogener erreicht. Die Steuerung gewährleistet einen sicheren und op-timalen Be-handlungsverlauf. Wenn das Befüllen mit Kohlendioxid vor dem Beheizen der Wände durchgeführt wird, so können unerwünschte thermische Gasbewegungen im Silo ver-mieden werden.

Das Kohlendioxid wird vorzugsweise von Vorratsflaschen über eine Gasaufberei-tungsheizung und eine Druckreduziereinheit dem Silo zugeführt. Am Ende der Be-handlung kann das Kohlendioxid an die Umgebung abgegeben werden. Um den Kohlendioxidverbrauch zu reduzieren wird aber vorzugsweise eine Rückgewinnung vorgesehen. Dazu wird die Gasleitung vom Anschluss am unteren Siloende über ein Rückgewinnungsventil mit einem Filter, einem daran anschliessenden Kompressor und einem Druckbehälter verbunden. Der Kompressor befüllt den Druckbehälter. Für eine weitere Behandlung kann nun das Kohlendioxid aus dem Druckbehälter über ein Wiedereinfüllventil in die Gasleitung mit dem Anschluss am unteren Si-loende und so ins Silo eingetragen werden.

Der Kompressor vorzugsweise zusammen mit dem Druckbehälter kann auch dazu verwendet werden bei der Behandlung im Silo zyklische Druckschwankungen zu er-zeugen. Dabei wird der Druck um wenige mbar erhöht und erniedrigt. Die tierischen Zellen werden so angeregt Kohlendioxid aufzunehmen und wieder abzugeben, was die Zellenaktivität anregt und die Behandlungsdauer verkürzt.

Um die Behandlung sicher und effizient durchzuführen ist eine Steuerung vorgese-hen, die mit den erwähnten Sensoren, mit dem Zirkulationsgebläse, der Zirkula-tionsheizung, gegebenenfalls einer Silowandheizung und bei automatisch betätigba-ren Schiebern und Ventilen auch mit diesen verbunden ist. Die Steuerung umfasst eine Bedienungseinrichtung und eine Anzeigeeinrichtung, um eine Behandlung aus-zulösen und den Zustand der Anlage bzw. die Behandlungssituation anzuzeigen. Vorzugsweise sind auch die Steuerelemente der Vorratsflaschen, der Gasaufberei-tungsheizung und der Dru ckreduziereinheit sowie insbesondere der Gasrückgewin-nung mit der Steuerung verbunden. Bei einer Ausführungsform mit Dichtigkeitskon-trolle sind auch die dafür nötigen steuerbaren Elemente mit der Steuerung verbunden.

Im Rahmen einer Standardbehandlung mit Kohlendioxid wird beispielsweise ca. 32 Tonnen Getreide im Silo mit 43m³ Inhalt mit Kohlendioxid möglichst vollständig um-geben und bei einer Umgebungstemperatur von beispielsweise 15°C auf 23°C bis 35°C er-wärmt. Die Anlage ist so ausgelegt, dass die Temperatur des Getreides in nützli-cher Zeit um 6°C bis 20°C erhöht werden kann. Die Heizleistung der Zirkula-tions-heizung und der Silowandheizung wird so gewählt, dass eine schonende Erwär-mung um 10°C in einer Zeit von 10 bis 30 Stunden erzielbar ist. Diese Heiz-leistung kann mit einer Silowandheizung mit einer Leistung von 8kW und einer Zir-kulations-heizung mit einer Heizleistung von 10kW bereitgestellt werden. Das Koh-lendioxid wirkt bereits während der Aufwärmphase von ca. einem Tag. Bei der erreichten Temperatur wird die Kohlendioxidbehandlung solange weitergeführt bis eine im Wesentlichen vollständige Vernichtung der Schädlinge er-zielt ist.

Bei einer Behandlungstemperatur von lediglich ca. 10°C ist eine Behandlungsdauer von 18 bis 24 Tagen zweckmässig. Bei einer Behandlungstemperatur von ca. 20°C ist eine Behandlungsdauer von 10 bis 14 Tagen sinnvoll. Bei im Wesentlichen 25°C genügen 5 bis 8 Tage, bei 30°C ca. 3 bis 5 Tage und bei 35° ca. 2 bis 3 Tage. Mit den beschriebenen Druckschwankungen kann die Behandlungszeit gegebenenfalls noch etwas reduziert werden.

Es versteht sich von selbst, dass mit der beschriebenen Anlage anstelle des Kohlendi-oxid auch andere Gase eingesetzt werden können. Dabei müssen einzelne Merkmale an die Eigenschaften der entsprechenden Gase angepasst werden. Wenn beispiels-weise ein Gas wie Stickstoff verwendet wird, das leichter ist als Luft, so ist eine Befül-lung von oben und ein gleichzeitiger Luftauslass unten zweckmässig. Dazu müssen le-diglich die Anschlüsse entsprechend angeordnet werden.

Die erfindungsgemässe Lösung ist nicht auf die Behandlung von körnigen Produkten beschränkt, sondern kann für die Behandlung aller Schüttgüter vorteilhaft eingesetzt werden. Insbesondere können auch Würfel oder Expandate, gegebenenfalls selbst Mehl, behandelt wer-den.

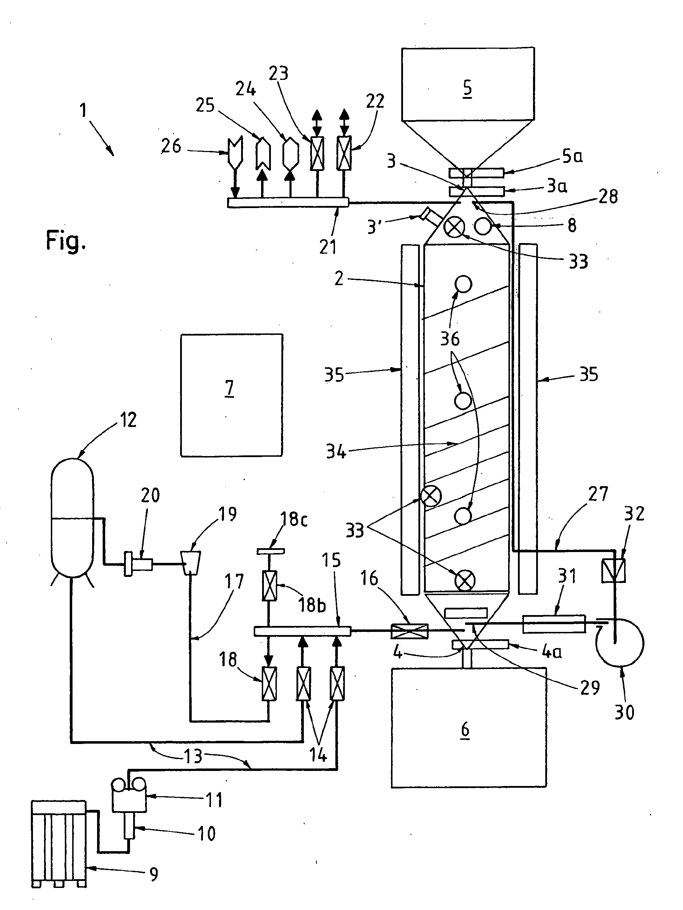

Die Zeichnungen erläutert die Erfindung anhand zweier Aus-füh-rungsbeispiele auf die sie aber nicht eingeschränkt ist. Dabei zeigt die Figur eine schematische Darstellung einer Behandlungsvorrichtung

Die dargestellte Ausführungsform der Vorrichtung 1 zum Behandeln von Schüttgut mit Kohlendioxid umfasst ein Silo 2 mit einer oberen Befüllöffnung 3 zum Einfüllen und einer unteren Austragsöffnung 4 zum Austragen von Schüttgut. An diesen beiden Öff-nungen sind ein oberer und ein unterer gasdichter Schieber 3a und 4a angeordnet. Die Schieber 3a und 4a sind vorzugsweise automatisch zu betätigen. In der darge-stellten Ausführungsform ist über dem Silo 1 ein Vordepot 5 und unter dem Silo 2 ein Nachdepot 6 angeordnet. Am unteren Ende des Vordepots 5 ist eine Öffnung mit einem Vordepotschieber 5a angeordnet. Gegebenenfalls umfasst das Silo 2 eine weitere gasdicht verschliessbare Befüllöffnung 3', um Schüttgut einzufüllen, das nicht aus dem Vordepot 5 ins Silo 2 eingetragen wird.

Um die Behandlung einfach und sicher durchführen zu können wird eine Steuerung 7 mit einer Bedienungseinrichtung, einer Anzeigeeinrichtung, Sensorverbindungen zu Sensoren und Steuerverbindungen zu Anlagekomponenten eingesetzt. Die Ver-bindung werden bei der Beschreibung der Komponenten bzw. ihrer Funktion er-wähnt, sind aber nicht eingezeichnet, weil sonst die Figur unübersichtlich würde.

Für eine Behandlung wird der untere Schieber 4a in die Schliessstellung gebracht und der Vordepotschieber 5a sowie der obere Schieber 3a in die Offenstellung ge-bracht, so dass Schüttgut aus dem Vordepot ins Silo 2 fliessen kann. Sobald die gewünschte Schüttgutmenge im Silo 2 ist, wird der obere Schieber 3a geschlossen. Mit mindestens einem Füllstandsensor 8 kann zumindest ein maximaler gegebe-nenfalls auch ein aktueller Füllstand erfasst werden. Für die aktuelle Füllstandser-fassung kann der Füllstandsensor 8 beispielsweise mit einer vom oberen Siloende aus nach unten durchgeführten Distanzmessung die Lage der Schüttgutoberfläche erfassen. Die Schieber 3a, 4a und der Füllstandsensor 8 sind vorzugsweise mit der Steuerung 7 verbunden, so dass die Betätigung und gegebenenfalls Lagekontrolle der Schieber 3a, 4a sowie die Füllstandsmessung von der Steuerung 7 durchgeführt werden kann. Die Steuerung 7 schliesst den oberen Schieber 3a vorzugsweise beim Erreichen eines vorgegebenen Füllstandes, sobald der Füllstand konstant bleibt oder nach einer vorgegebenen Befüllzeit.

Das für die Behandlung des Schüttgutes benötigte Kohlendioxid gelangt von Vorrats-flaschen 9 über eine Gasaufbereitungsheizung 10 und eine Druckreduziereinheit 11 oder von einem Druckbehälter 12 über entsprechende Zuführleitungen 13 und je ein Zuführventil 14 in eine Gasleitung 15. Die Gasleitung 15 ist über ein Befüllventil 16 mit dem unteren Endbereich des Innenraums des Silos 2 verbunden. Vorrichtungen mit einem Druckbehälter 12 werden eingesetzt, wenn das Kohlendioxid nach einer Schütt-gutbehandlung für eine weitere Behandlung in den Druckbehälter geführt wird. Für diese Rückführung ist eine Rückführleitung 17 mit einem Rückführventil 18 an der Gasleitung 15 angeschlossen. In der Rückführleitung 17 ist ein Filter 19 und ein Kom-pressor 20 angeordnet, so dass das Kohlendioxid frei von Schüttgutpartikeln unter Druck in den Druckbehälter 12 eingefüllt werden kann. Wenn auf eine Gasrückgewin-nung verzichtet wird, kann ein Gasauslasselement 18c über ein Gasaustrittsventil 18b mit der Gasleitung 15 verbunden werden.

Um ein kontrolliertes Ein- und Austragen von Kohlendioxid zu gewährleisten werden die Zuführventile 14, das Befüllventil 16 und das Rückführventil 18 von der Steuerung 7 geregelt. Bei der Zuführung von Kohlendioxid aus den Vorratsflaschen 9 wird vorzugs-weise auch die Gasaufbereitungsheizung 10 und die Druckreduziereinheit 11 von der Steuerung 7 geregelt.

Weil beim Eintragen von Kohlendioxid ins Silo 2 Luft aus dem Silo 2 verdrängt werden soll, ist eine Ausgleichsleitung 21 mit Auslassventil 22 am oberen Siloende ange-schlossen. Gegebenenfalls ist zusätzlich zum Auslassventil 22 noch ein weiteres Auslassventil 23 an der Ausgleichsleitung 21 angeordnet, das beim Auftreten eines Überdruckes im Silo 2 geöffnet werden kann. Um einen Überdruck im Silo 2 erfas-sen zu können, ist ein Druckfühler 24 an der Ausgleichsleitung 21 angeschlossen. Die Auslassventile 22 und 23 sowie der Druckfühler 24 sind mit der Steuerung 7 ver-bunden, bzw. von dieser zu betätigen. Wenn die Steuerung 7 ausfällt wird das Silo 2 durch eine Überdrucksicherung 25 und eine Unterdrucksicherung 26 vor uner-wünschten Druckänderungen geschützt.

Eine Zirkulationsleitung 27 führt von einem oberen Anschluss 28 ans Siloinnere zu einem unteren Anschluss 29 ans Siloinnere. In der Zirkulationsleitung 27 ist ein Zir-kulationsgebläse 30, eine Zirkulationsheizung 31 und ein Richtungsventil 32 ange-ordnet ist. Das Zirkulationsgebläse 30 erzeugt eine Gaszirkulation durch die Zirkulati-onsleitung 27 von oben nach unten und im Silo 2 durch das Schüttgut von unten nach oben. Bei eingeschaltetem Gebläse kann das zirkulierende Gas von der Zirkulations-heizung 31 aufgeheizt werden, so dass das erwärmte Gas beim Durchströmen des Schüttgutes das Schüttgut aufheizt. Wenn das Gebläse 30 nicht eingeschaltet ist, kann das Richtungsventil 32 eine Strömung von unten nach oben durch die Zirkulationslei-tung verhindern. Das Richtungsventil 32 ist vorzugsweise ein Einweg- oder Rück-schlagventil, es könnte gegebenenfalls aber auch ein gesteuertes Schliessventil einge-setzt werden.

Die Zirkulationsheizung ist vorzugsweise als elektrische Heizung ausgebildet. Es ver-steht sich von selbst, dass die Heizungsart der benötigte Heizleistung entsprechen muss. Bei Vorrichtungen, die eine grosse Heizleistung benötigen, ist gegebenenfalls eine Heizvorrichtung mit Brenner, insbesondere ein Gasbrenner, zweckmässig. Bei Anlagen, in denen unbenutzte Prozesswärme vorhanden ist, kann auch diese verwen-det werden. Für die Übertragung der Heizwärme an das zirkulierende Gas wird vor-zugsweise ein Wärmetauscher eingesetzt. Gegebenenfalls ist die Heizung mit einem Wärmespeicher und/oder einem Übertragungskreislauf verbunden, von dem die Wärme gesteuert an das Gas übertragen werden kann. Um bei einem trägen Wärmetauscher die Erwärmung des zirkulierenden Gases schnell ausschalten zu können, ist parallel zum Wärmetauscher gegebenenfalls ein nicht beheizter Bypass ausgebildet, wobei Ventile regeln, ob der Bypass oder der Wärmetaucher vom Gas durchströmt wird. Für grosse Heizleistungen werden gegebenenfalls weitere Zirkulationsleitungen eingesetzt, welche die unteren Anschlüsse 29 auf verschiedenen Höhen des Silos 2 haben. Wenn das Gas von einem Zirkulationsgebläse an zwei oder mehr unteren Anschlüssen 29 eingetragen wird, kann mit Durchflussregelungen gewährleistet werden, dass an allen Anschlüssen gewünschte Gasmengen eingetragen werden, was aufwändig und daher wenig vorteilhaft ist.

Um die Zirkulation und die Zirkulationsheizung zu regeln, sind das Gebläse 30, die Zir-kulationsheizung 31 und mindestens ein Temperatursensor 33 an der Steuerung 7 an-geschlossen. Mit dem mindestens einen Temperatursensor 33 wird die Temperatur im Silo 2, vorzugsweise zumindest im Nahbereich über dem unteren Anschluss 29 der Zirkulationsleitung 27, insbesondere aber auch am oberen Endbereich des Silos 2, erfasst. Beim Überschreiten einer vorgegebenen maximalen Temperatur wird die Heizleistung der Zirkulationsheizung 31 reduziert, insbesondere ausgeschaltet. Beim Unterschreiten einer vorgegebenen minimalen Temperatur wird die Heizleis-tung der Zirkulationsheizung 31 erhöht. Bei ausgeschalteter Zirkulationsheizung 31 und einer Temperaturverteilung im Schüttgut mit Temperaturen, die bei allen Tem-peratursensoren 33 über einem vorgegebenen minimalen Wert liegen, wird vor-zugsweise auch das Zirkulationsgebläse 30 ausgeschaltet.

Am Silo 2 ist eine Silowandheizung 34 angeordnet. Die beheizte Silowand erwärmt das an die Silowand angrenzende Schüttgut. Die Silowandheizung 34 ist vorzugsweise elektrisch betreibbar und umfasst mindestens eine Heizmatte oder mindestens ein Heizband, wobei gegebenenfalls am unteren Bereich des Silos eine grössere Heiz-leistung installiert ist als am oberen. Wenn die Wandheizung auf verschiedenen Niveaus getrennt eingeschaltet werden kann, so erlaubt dies ein gezieltes Heizen im Bereich mit Schüttgut, bzw. mit Schüttgut das noch nicht die gewünschte Tempe-ratur aufweist.

Die Steuerung 7 ist mit mindestens einem weiteren Temperatursensor 33 verbunden, der die Temperatur im Nahbereich der Silowand erfasst. Beim Überschreiten einer vorgegebenen maximalen Temperatur beim wandnahen Sensor wird die Heizleis-tung der Silowandbeheizung reduziert oder gegebenenfalls die Silowandbeheizung ausgeschaltet. Beim Unterschreiten einer vorgegebenen minimalen Temperatur wird die Heizleistung der Silowandbeheizung erhöht.

Um den Wärmeverlust des Silos 2 klein zu halten wird das Silo 2 mit einer Siloiso-lierung 35 umhüllt. Vorzugsweise wird auch zumindest ein Teil der Zirkulationslei-tung 27 von der Siloisolierung 35 umschlossen. Gegebenenfalls wird aber auch die Zirkulationsleitung 27, insbesondere im Bereich der Zirkulationsheizung 31 vom Zir-kulationsgebläse 30 zum unteren Anschluss 29, mit einer eigenen Isolierung iso-liert. Um gegebenenfalls eine direkt ablesbare Temperaturinformation bereitzustel-len, werden auf verschiedenen Höhen Temperaturmanometer angeordnet, welche Temperaturen im Schüttgut anzeigen.

Um nach dem Verschliessen der Befüllöffnung 3 eine Dichtigkeitsprüfung durchzufüh-ren, werden die Auslassventile 22 und 23 verschlossen. Durch die Gasleitung 15 wird Gas ins Silo 2 eingetragen bis ein vorgegebener Überdruck im Silo 2 aufgebaut ist und nach dem Verschliessen des Befüllventils 16 wird der Druck im Silo 2 über eine vorge-gebene Zeit mit dem Druckfühler 24, der vorzugsweise auch an die Steuerung 7 an-geschlossen ist, überwacht. Bei einem genügend guten Druckerhalt wird das Silo 2 als gasdicht verschlossen erkannt und für eine Behandlung freigegeben.

Der Kompressor 20 kann zusammen mit dem Druckbehälter 12 dazu verwendet werden, bei der Behandlung im Silo 2 zyklische Druckschwankungen zu erzeugen. Der Druck im Silo 2 wird beispielsweise um einige mbar erhöht und erniedrigt. Dazu wird Kohlendioxid unter Druck über die Zuführleitungen 13, das geöffnete Zuführ-ventil 14 und das geöffnete Befüllventil 16 ins Silo 2 eingetragen. Mit dem Druckfühler 24 kann das Erreichen des vorgegebenen Druckanstieges erfasst und über die Steue-rung 7 das Zuführventil 14 geschlossen werden. Zum Reduzieren des Druckes im Silo 2 wird nun das Rückführventil 18 geöffnet und mit dem Kompressor 20 Kohlendi-oxid aus dem Silo 2 ausgetragen bis der Druckfühler 24 den gewünschten Druckabfall ermittelt. Jetzt kann das Rückführventil 18 geschlossen und der Zyklus von vorne be-gonnen werden. Es versteht sich von selbst, dass die Behandlung mit Druckschwankun-gen auch mit der Druckreduktion begonnen werden kann. Die tierischen Zellen werden durch die Druckschwankungen ange-regt Kohlendioxid aufzunehmen und wieder abzugeben, was die Zellenaktivität an-regt und die Behandlungsdauer verkürzt.

## Patentansprüche

1. Verfahren zum Behandeln von Schüttgut mit Kohlendioxid in einem Silo (2) mit einer oberen Befüllöffnung (3) zum Einfüllen und einer unteren Austragsöffnung (4) zum Austragen von Schüttgut, einer Gasleitung (15) mit Ventil (16) zum Zufüh-ren von Kohlen-dioxid und einer Zirkulationsleitung (27) von einem oberen zu einem unteren An-schluss (28, 29) ans Siloinnere mit einem Zirkulationsgebläse (30), wobei am Anfang einer Behandlung Schüttgut eingefüllt und die obere Befüll-öffnung (3) gas-dicht verschlossen wird , **dadurch** ge-kenn-zeichnet, dass anschliessend Kohlendi-oxid von Vorratsflaschen (9) über eine Gasaufbereitungs-heizung (10) und eine Druckre-duziereinheit (11) oder von einem Druckbehälter (12) durch die Gasleitung (15) ins Silo (2) einge-tragen sowie Luft durch eine am oberen Siloende angeschlossene mit der Umge-bung verbundene Ausgleichs-leitung (21) mit Ventil (22) abgelassen wird, wobei zumindest beim Eintragen von Kohlendioxid das Durchströmen der Zirkulationsleitung (27) von unten nach oben durch ein Ventil (32) verhindert wird, nach dem Befüllen des Silos (2) mit Kohlen-dioxid die Gasleitung (15) und die Ausgleichsleitung (21) gasdicht ver-schlossen werden, zum Fördern von Gas durch die Zirkulationsleitung (27) und das Schüttgut das Zirkulationsgebläse (30) eingeschaltet wird und zum Erwärmen des ge-förderten Gases eine Zirkulationsheizung (31) eingeschaltet wird, so dass das er-wärmte Gas das Schüttgut erwärmt bis das Schüttgut auf Temperaturen erwärmt ist, welche über einer vorgegebenen minimalen Behandlungstemperatur liegen.

2. Verfahren nach Anspruch 1, **dadurch gekennzeichnet, dass** nach dem Verschlies-sen der Befüllöffnung (3) eine Dichtigkeitsprüfung durchgeführt wird, wobei vorzugs-weise das Ventil (22) der Ausgleichsleitung (21) verschlossen sowie durch die Gasleitung (15) Gas ins Silo (2) eingetragen wird bis ein vorgegebener Über-druck im Silo (2) aufgebaut ist und nach dem Verschliessen des Ventils (16) der Gasleitung (15) über eine vorgegebene Zeit der Druck im Silo (2) überwacht wird.

3. Verfahren nach Anspruch 1 oder 2, **dadurch gekennzeichnet, dass** zum weiteren Erwärmen des Schüttgutes die Silowand beheizt wird, vorzugsweise elektrisch mit mindestens einer Heizmatte oder mindestens einem Heizband, wobei gege-benenfalls am unteren Bereich des Silos mehr Wärme eingetragen wird als am oberen und insbesondere die Wandheizung (34) auf verschiedenen Niveaus getrennt eingeschaltet wird.

4. Verfahren nach einem der Ansprüche 1 bis 3, **dadurch gekennzeichnet, dass** mit min-destens einem Temperatursensor (33) die Temperatur im Silo (2), vorzugsweise zumin-dest im Nahbereich über dem unteren Anschluss (29) der Zirkulationslei-tung (27) insbe-sondere aber auch am oberen Endbereich des Silos (2), erfasst und beim Über-schreiten einer vorgegebenen maximalen Temperatur die Heiz-leistung der Zir-kulationsheizung (31) reduziert, oder gegebenenfalls die Zirkula-tionsheizung (31) ausge-schaltet und beim Unterschreiten einer vorgegebenen minimalen Temperatur die Heizleistung der Zirkulationsheizung (31) erhöht wird, wobei bei ausgeschalteter Zirkulationsheizung (31) und einer Temperatur-verteilung im Schüttgut mit Tempera-turen, die bei allen Temperatursensoren (33) über einem vorgegebenen minimalen Wert liegen vorzugsweise auch das Zirkulationsgebläse (30) ausgeschaltet wird.

5. Verfahren nach Anspruch 4, **dadurch gekennzeichnet, dass** mit mindestens einem weiteren Temperatursensor (33) die Temperatur im Nahbereich der Silowand er-fasst und beim Überschreiten einer vorgegebenen maximalen Temperatur die Heiz-leistung der Wandheizung (34) reduziert, oder gegebenenfalls die Wand-heizung (34) ausgeschaltet wird und beim Unterschreiten einer vorgegebenen mini-malen Temperatur die Heizleistung der Wandheizung (34) erhöht wird.

6. Verfahren nach einem der Ansprüche 1 bis 5, **dadurch gekennzeichnet, dass** nach einer vorgegebenen Behandlungszeit ab dem Einfüllen des Kohlendioxides, oder ab dem Erreichen der vorgegebenen minimalen Temperatur im überwiegenden Anteil des Schüttgutes, bei ausgeschaltetem Zirkulationsgebläse (31) Ventile (16, 22) der Gaslei-tung (15) und der Ausgleichsleitung (21) geöffnet werden und das Kohlen-dioxid von der Gasleitung (15) über eine Auslasseinrichtung (18b, 18c) an die Um-gebung abgegeben, vorzugs-weise aber über einen Filter (19) und einen Kompres-sor (20) einem Druckbehälter (12) zugeführt wird.

7. Vorrichtung zum Behandeln von Schüttgut mit Kohlendioxid mit einem Silo (2) mit einer oberen Befüllöffnung (3) zum Einfüllen und einer unteren Austragsöffnung (4) zum Austragen von Schüttgut, einer Gasleitung (15) mit Ventil (16) zum Zufüh-ren von Kohlendi-oxid und einer Zirkulationsleitung (27) von einem oberen zu einem unteren An-schluss (28, 29) ans Siloinnere mit einem Zirkulationsgebläse (30), **dadurch** ge-kenn-zeichnet, dass Kohlendioxid Vorratsflaschen (9) über eine Gasaufbereitungsheizung (10) und eine Druckreduziereinheit (11) oder ein Druckbehälter (12) an die Gasleitung (15) angeschlossen sind, eine Ausgleichs-leitung (21) mit Ventil (22) am oberen Siloende angeschlossen ist, in der Zirkula-tionsleitung (27) ein Ventil (32) angeordnet ist, um eine Strömung von unten nach oben ausschliessen zu können, und an der Zirkulationsleitung (27) eine Zirkulati-onsheizung (31) zum Erwärmen des geförderten Gases angeordnet ist.

8. Vorrichtung nach Anspruch 7, **dadurch gekennzeichnet, dass** eine Wandheizung (34) zum Erwärmen des Schüttgutes an der Silowand angeordnet ist, welche vor-zugsweise elektrisch betreibbar ist und mindestens eine Heizmatte oder min-destens ein Heizband umfasst, wobei gegebenenfalls am unteren Bereich des Silos (2) eine grössere Heizleistung installiert ist als am oberen und, insbeson-dere die Wandheizung (34) auf verschiedenen Niveaus getrennt eingeschaltet werden kann.

9. Vorrichtung nach Anspruch 7 oder 8, **dadurch gekennzeichnet, dass** eine Steue-rung (7) eine Bedienungseinrichtung, eine Anzeigeeinrichtung, Sensorverbin-dun-gen zu Sensoren (33, 24) und Steuerverbindungen zu Anlagekomponenten (3a, 4a, 8, 10, 14, 16, 18, 20, 22, 23, 24, 30, 31, 33) umfasst, um mit mindes-tens einem Temperatursensor (33) die Temperatur im Silo (2), vorzugsweise zu-mindest im Nahbereich über dem unteren Anschluss (29) der Zirkulationsleitung (27), insbeson-dere aber auch am oberen Endbereich des Silos (2), zu erfassen, beim Über-schreiten einer vorgegebenen maximalen Temperatur die Heizleis-tung der Zir-kulationsheizung (31) zu reduzieren, insbesondere auszuschalten, beim Unter-schreiten einer vorgegebenen minimalen Temperatur die Heizleistung der Zir-kulationsheizung (31) zu erhöhen, und bei ausgeschalteter Zirkulationsheizung (31) und einer Temperaturverteilung im Schüttgut mit Tem-peraturen, die bei allen Tem-peratursensoren (33) über einem vorgegebenen minimalen Wert liegen, vorzugs-weise auch das Zirkulationsgebläse (30) auszu-schalten.

10. Vorrichtung nach Anspruch 9, **dadurch gekennzeichnet, dass** die Steuerung (7) mit min-destens einem weiteren Temperatursensor (33) verbunden ist, um die Tempe-ratur im Nahbereich der Silowand zu erfassen und beim Überschreiten einer vor-gege-benen maximalen Temperatur die Heizleistung der Wandheizung (34) zu redu-zieren, oder gegebenenfalls die Wandheizung (34) auszuschalten und beim Unterschreiten einer vorgegebenen minimalen Temperatur die Heizleistung der Wandheizung (34) zu erhöhen.

11. Vorrichtung nach Anspruch 9 oder 10, **dadurch gekennzeichnet, dass** die Gaslei-tung (15) je über ein Ventil (18, 18b) mit einer Auslasseinrichtung (18c) an die Um-gebung, vorzugs-weise aber über einen Filter (19) und einen Kompressor (20) mit einem Druckbehälter (12) sowie direkt mit dem Druckbehälter (12) verbunden ist und insbesondere die Steuerung (7) die Ventile der Gasleitung (15) und der Aus-gleichsleitung (21) betätigbar macht, um nach einer vorgegebenen Behandlungszeit ab dem Einfüllen des Kohlendioxides, oder ab dem Erreichen der vorgegebenen minimalen Temperatur im überwiegenden Anteil des Schüttgutes, bei ausgeschal-tetem Zirkulationsgebläse (30) die Ventile der Gasleitung (15) und der Ausgleichs-leitung (21) zu öffnen und das Kohlendioxid von der Gasleitung (15) über die Aus-lasseinrichtung (18b, 18c) an die Umgebung abzugeben, vorzugsweise aber dem Druckbehälter (12) zuzuführen.
